# EUROPÄISCHE PATENTANMELDUNG

(11) **EP 1 159 958 A1**
(43) Veröffentlichungstag der Anmeldung: **05.12.2001**
(21) Anmeldenummer: 00118143.7
(22) Anmeldetag: 29.08.2000
(51) Int. Cl.: A61K 31/01, A61K 9/00, A61K 47/06, A61P 37/08, A61P 11/02

(54) **Nasensalbe auf Basis von Vaselin**

(30) Priorität: 31.05.2000 DE 20009841 U
(71) Anmelder: Phyt-Immun GmbH, 66424 Homburg (DE)
(72) Erfinder: Theiss, Peter, Dr., 66424 Homburg/Saar (DE)
(74) Vertreter: Ricker, Mathias, Dr.Dipl.-Chem.

(57) **Zusammenfassung**

Nasensalbe zur Prophylaxe von inhalationsallergischen Reaktionen umfassend mindestens ein Gemisch aus gesättigten Kohlenwasserstoffen, die mindestens ein Gemisch aus gesättigten Kohlenwasserstoffen mit einer Viskosität nach DIN 51 562 von > 6 mm²/s (100 °C) besitzt.

## Beschreibung

Die vorliegende Erfindung betrifft eine Nasensalbe umfassend bei Raumtemperatur gelartige Gemische überwiegend gesättigter Kohlenwasserstoffe, sowie deren Verwendung zur Prophylaxe von inhalationsallergischen Reaktionen, insbesondere der atopischen Rhinitis in der Erscheinungsform "Heuschnupfen". In einer bevorzugten Ausführungsform umfaßt die erfindungsgemäße Nasensalbe noch mindestens einen für Salben geeigneten pflegenden Zusatzstoff.

In den westlichen Industriezivilisationen sind Inhalationsallergien mittlerweile stark verbreitet, wobei die Tendenz weiterhin ansteigt. Besonders sind es dabei die durch Pollen, Pilzsporen, verschiedene Stäube (z.B. von Holz, Mehl oder Hausstaub), chemische oder tierische (Federn, Haare) Reizstoffe inhalativ ausgelösten Formen der atopischen Rhinitis, die mit ihren Begleiterscheinungen das Befinden des Patienten bis zur Arbeitsunfähigkeit beeinträchtigen kann.

Das quälende Jucken von Nase, Pharynx, Rachendach und Augen, dem Tränenfluß, Niesen und wäßrige Sekretion aus der Nase folgen, ist häufig begleitet von Kopfschmerz, Reizbarkeit, Appetitlosigkeit, Depressionen und Schlaflosigkeit. Im weiteren Verlauf können sich Hustenanfälle und asthmatisches Keuchen entwickeln.

Als saisonale, durch Pollen von Bäumen, Sträuchern, Gräsern sowie Wild- oder Zierpflanzen ausgelöste Reaktion steht unter den atopischen Rhinitiden der sogenannte "Heuschnupfen" an erster Stelle.

Die meisten der bislang angewendeten therapeutischen und prophylaktischen Maßnahmen zur Kontrolle oder Ausschaltung inhalationsallergischer Reaktionen weisen mehr oder weniger schwerwiegende Nachteile in Bezug auf Kosten, Effizienz, gesundheitliche Nebenwirkungen und Anwendungskomfort auf.

So sind Kuren, in denen Patienten in pollenarme Gebiete, wie Hochgebirge oder vegetationsarme Inseln während der Pollensaison für mehrere Wochen aus dem Expositionsgebiet entfernt werden oder aber ein Wohnungs- oder Berufswechsel zur Vermeidung einer Exposition für zahlreiche Patienten nur schwer zu verkraften.

Durch diese Karenz versucht man eine weitgehende Entfernung der krankheitsauslösenden Allergene aus der Umgebung des Kranken zu errreichen.

Die vollständige Entfernung inhalativer Allergene ist nicht möglich. Es gibt lediglich die Chance, die Kontaktflächen zum Allergen, also die Schleimhaut als Reaktionsort auszuschalten. Dies wird als Teilkarenz beschrieben.

Der Einbau von Allergen-Filtern in Belüftungs- und Klimaanlagen von Wohnungen, Arbeitsstätten und Autos kann nur die Exposition in diesen speziell geschützten Bereichen verhindern und ist ebenfalls mit nicht vernachlässigbaren Kosten verbunden.

Das Tragen eines Atemfilters über Mund und Nase ist kostengünstig und wirksam, aber unbequem.

Die Desensibilisierung (Hyposensibilisierung, Immunisierung) erfordert aufwendige Tests für die Wahl eines wirksamen Antigen-Extrakts. Ob eine klinische Besserung durch die Behandlung eintritt, ist dennoch nicht sicher vorhersagbar. Dieses Verfahren ist außerdem nicht frei von der Gefahr gesundheitlich bedenklicher Begleiterscheinungen, wie zum Beispiel asthmatoide und urtikarielle Beschwerden. Ferner erfordern die Desensibilisierungsbehandlungen einen verhältnismäßig großen Kosten- und Zeitaufwand.

Die symptomatische Behandlung der allergischen Reaktion mit oralen Antihistaminika zur Schleimhautabschwellung ist bei vielen dieser Präparate mit dem Risiko einer sedierenden Wirkung behaftet, die sich auf das Wohlbefinden und die Reaktionsfähigkeit des Patienten negativ auswirkt. Es treten weiterhin nicht selten Nebenwirkungen, wie zum Beispiel Kopfschmerzen, Mundtrockenheit und Konzentrationsstörungen auf.

Ferner ist auch die Anwendung von Sympatikomimetika, Präparate auf Cromoglicinsäure-Basis und von Glukokortikoiden wegen zahlreicher Nebenwirkungen und/oder hoher Kosten mit Nachteilen behaftet.

Bezüglich einer detaillierten Darstellung zu diesem Stand der Technik wird auf die DE-C 41 17 887 verwiesen. Diese bezieht sich auf die Verwendung von pharmazeutisch/kosmetisch als Vaselin definierten gesättigten Kohlenwasserstoffen als Nasensalbe zur Prophylaxe von inhalationsallergischen Reaktionen, insbesondere vom Typ "Heuschnupfen", wobei dort ein Vaselin mit folgenden Eigenschaften verwendet wurde:
- Erstarrungspunkt nach DIN 51 556: 49 bis 52 °C;
- Viskosität nach DIN 51 562: 6 mm²/s (100 °C);
- Konuspenetration nach DIN 51 580: 150 bis 170;
- mittlere Kettenlänge der Kohlenwasserstoffe des Vaselins: 26 ± 1 C-Atome;
- C-Zahlbereich der Kohlenwasserstoffe: C₁₅ bis C₆₀.

In Anbetracht dieses Standes der Technik lag der vorliegenden Erfindung die Aufgabe zugrunde, eine weiter verbesserte Nasensalbe zur wirkungssicheren Prophylaxe von inhalationsallergischen Reaktionen auf der Basis eines weiter verbesserten Vaselins bereitzustellen. Diese Nasensalbe sollte wiederum für den Patienten keine gesundheitlichen Risiken beinhalten, ein breites Wirkungsspektrum gegen verschiedene Allergene der Atemluft aufweisen, angenehm anzuwenden sein und schlußendlich nur geringe Behandlungskosten verursachen.

Diese und weitere Aufgaben werden durch die Nasensalbe gemäß Patentanspruch 1 gelöst, nämlich durch eine Nasensalbe zur Prophylaxe von inhalationsallergischen Reaktionen umfassend mindestens ein Gemisch aus gesättigten Kohlenwasserstoffen.

Im Gegensatz zu dem in DE-C 41 17 887 beschriebenen Vaselin weist das nunmehr erfindungsgemäß verwendete Vaselin einen größeren Anteil an langkettigen gesättigten Kohlenwasserstoffen auf.

Bezüglich des erfindungsgemäß verwendbaren Vaselins existieren keinerlei Beschränkungen, solange dieses den Reinheitsanforderungen der pharmazeutisch/kosmetisch verwendbaren Qualität gemäß Reinheitsvorschrift DAB entspricht und die erfindungsgemäß erforderlichen Parameter bezüglich der üblichen Eigenschaften aufweist.

So weist das erfindungsgemäß verwendete Vaselin eine Viskosität bei 100 °C nach DIN 51 562 von > 6 mm²/s, vorzugsweise > 8 mm²/s und insbesondere eine Viskosität von 8,5 bis 15 mm²/s auf.

In einer weiteren bevorzugten Ausführungsform wird ein Vaselin verwendet, daß mindestens eine der folgenden Eigenschaften (a) bis (e) aufweist:
(a) Erstarrungspunkt (DIN 51 556) 47 bis 56 °C
(b) Konuspenetration nach DIN 51 580 von 155 bis 185;
(c) maximal 5 Gew.-% der gesättigten Kohlenwasserstoffe haben eine Kettenlänge von ≤ 25 ± 2;
(d) C-Zahlbereich der gesättigten Kohlenwasserstoffe (Bestimmung mittels GC): umfaßt C₁₉ bis C₅₂, jeweils ± 2; und
(e) Zahlenmittel des Molekulargewichts: >400, vorzugsweise >480 und insbesondere > 500 g/mol.

Als für Salben pflegende Zusatzstoffe können alle für diesen Zweck bekannten Zusatzstoffe eingesetzt werden, wobei es sich vorzugsweise um Silikone, Panthenol, Lanolin, Lecithin, Dexpanthenol, Provitamin B5, Pantothensäure, Panthenylethylether, Panthenyl-ethylether-acetat, Panthenyltriacetat, Allantoin, pflanzliche (Algen, Flechten) Öle, wie z.B. Mandelöl, Weizenkeimöl, Avocadoöl, Wachse, wie z.B. Paraffine, Extrakte, vorzugsweise CO₂-Extrakte, wie z.B. Kamille, Sanddorn und Calendula handelt.

Das Auftragen der erfindungsgemäßen Nasensalbe kann mit dem Finger oder mit einer Applikationshilfe erfolgen. Für die Schutzwirkung gegen inhalationsallergische Reaktionen ist aber der Ort des Auftrags in der Nase wesentlich. Damit eine sichere Prophylaxe erreicht wird, muß gewährleistet sein, daß im Bereich der beiden Nasenklappen die Naseninnenwände mit der Nasensalbe bedeckt sind.

Wird die erfindungsgemäße Nasensalbe nur am unteren Ende des Nasenvorhofs im Bereich der äußeren Nasenlöcher aufgetragen, so kann die inhalationsallergische Reaktion nicht ausgeschlossen werden. Bei richtiger Anwendung ist es jedoch mit Hilfe der erfindungsgemäßen Nasensalbe möglich, eine Teilkarenz für inhalative Allergene zu erreichen. Da die erfindungsgemäße Nasensalbe aufgrund ihrer besonderen physikalischen Eigenschaften nicht in die Nasenschleimhäute eindringen kann und nicht resorbiert wird, sondern sich als Schutzfilm auflegt, entsteht eine mechanische Barriere, für die mit dem Atemstrom aufgenommenen und transportierten Allergenträger. Dadurch kann die allergische Reaktion weitestgehend verhindert werden.

Wie bereits in der DE-C 41 17 887 beschrieben, besteht ein wesentlicher Vorteil der vorliegenden Erfindung darin, daß allergische Reaktionen ausbleiben, ohne daß der Stoffwechsel des Patienten durch systemische Wirkstoffe belastet werden muß. Die Hauptbestandteile der Nasensalbe, die erwähnten gesättigten Kohlenwasserstoffe verhalten sich inert und werden nicht resorbiert. Damit ist die erfindungsgemäße Nasensalbe risikolos einsetzbar. Darüber hinaus kommt es auch nicht zu einem Nachlassen der Wirkung durch Gewöhnung des Patienten-Organismus. Die Geruchs- und die damit gekoppelte Geschmacksempfindungsfähigkeit bleiben unbeeinträchtigt.

Als weitere Vorteile der erfindungsgemäßen Nasensalbe sind zu nennen:
- vergleichsweise geringe Kosten;
- Patient kann die vorbeugende Behandlung selbst durchführen;
- die Applikation der erfindungsgemäßen Nasensalbe mit Zusatz hat gleichzeitig eine pflegende Wirkung.

Darüber hinaus ist die erfindungsgemäße Nasensalbe nicht nur in der Lage, die Schwellung, Sekretabsonderung und den Juckreiz im Naseninneren bei Allergen-Exposition zu verhindern, sondern auch die Reizreaktionen im Augen- und im Halsbereich auszuschalten.

Demgemäß betrifft die vorliegende Erfindung auch die Verwendung mindestens eines pharmazeutisch/kosmetisch als Vaselin definierten Gemischs aus gesättigten Kohlenwasserstoffen, wie in einem der Ansprüche 1 bis 5 definiert, in bzw. als Nasensalbe(n) zur Prophylaxe von inhalationsallergischen Reaktionen, wobei insbesondere die atopische Rhinitis vom Typ "Heuschnupfen" positiv beeinflußt werden kann.

Abschließend sei noch erwähnt, daß aufgrund der hohen Effizienz der erfindungsgemäßen Nasensalbe bzw. der erfindungsgemäßen Verwendung eine lang anhaltende, zuverlässige Schutzwirkung erreicht wird, obwohl man mit sehr kleinen Eintragmengen auskommt. Mit Mengen von wenigen Milligramm, die vor den Nasenklappen verstrichen werden, kann selbst bei starkem Pollenflug über mehrere Stunden hinweg Beschwerdefreiheit erzielt werden, ohne daß ein erneutes Auftragen der erfindungsgemäßen Nasensalbe notwendig wäre.

## Patentansprüche

1. Nasensalbe zur Prophylaxe von inhalationsallergischen Reaktionen umfassend mindestens ein Gemisch aus gesättigten Kohlenwasserstoffen
**dadurch gekennzeichnet, daß**
das mindestens eine Gemisch aus gesättigten Kohlenwasserstoffen eine Viskosität nach DIN 51 562 von > 6 mm²/s (100 °C) besitzt.

2. Nasensalbe nach Anspruch 1, wobei das mindestens eine Gemisch aus gesättigten Kohlenwasserstoffen weniger als 20 Gew.-% lineare gesättigte Kohlenwasserstoffe (n-Paraffine) aufweist.

3. Nasensalbe nach Anspruch 1 oder 2, wobei das mindestens eine Gemisch aus gesättigten Kohlenwasserstoffen mindestens eine der folgenden Eigenschaften aufweist:
(a) Erstarrungspunkt (ISO 2207) 47 bis 56 °C
(b) Konuspenetration nach DIN 51 580 von 155 bis 185;
(c) maximal 5 Gew.-% der gesättigten Kohlenwasserstoffe haben eine Kettenlänge von ≤ 25 ± 2;
(d) C-Zahlbereich der gesättigten Kohlenwasserstoffe (Bestimmung mittels GC): umfaßt C₁₉ bis C₅₂, jeweils ± 2; und
(e) Zahlenmittel des Molekulargewichts: >400, vorzugsweise >480 und insbesondere > 500 g/mol.

4. Nasensalbe nach einem der Ansprüche 1 bis 3, das zusätzlich mindestens einen pflegenden Zusatzstoff enthält.

5. Nasensalbe nach Anspruch 4, wobei der pflegende Zusatzstoff ausgewählt wird unter Silikonen, Panthenol, Lanolin, Lecithin, Dexpanthenol, Provitamin B5, Pantothensäure, Panthenylethylether, Panthenylethylether-acetat, Panthenyltriacetat, Allantoin, Mandelöl, Weizenkeimöl, Avocadoöl, Paraffinen und CO₂-Extrakten von Kamille, Sanddorn oder Calendula, sowie Gemischen aus zwei oder mehr davon.

6. Verwendung mindestens eines pharmazeutisch/kosmetisch als Vaselin definierten Gemischs aus gesättigten Kohlenwasserstoffen, wie in einem der Ansprüche 1 bis 5 definiert, in oder als Nasensalbe(n) zur Prophylaxe von inhalationsallergischen Reaktionen.

7. Verwendung nach Anspruch 6, wobei es sich hier bei der inhalationsallergischen Reaktion um eine atopische Rhinitis vom Typ "Heuschnupfen" handelt.
